# EUROPEAN PATENT APPLICATION

(11) **EP 4 844 390 A1**
(43) Date of publication of application: **25.11.2026**
(21) Application number: 24872578.0
(22) Date of filing: 27.09.2024
(51) Int. Cl.: G01N 27/02, C12M 1/34, C12Q 1/02

(54) **ELECTRIC TOMOGRAPHY METHOD AND ELECTRIC TOMOGRAPHY DEVICE**

(30) Priority: 29.09.2023 JP 2023170589
(71) Applicant: National University Corporation Chiba University, Chiba-shi, Chiba 263-8522 (JP)
(72) Inventor: KAWASHIMA, Daisuke, Chiba-shi, Chiba 263-8522 (JP); LI, Songshi, Chiba-shi, Chiba 263-8522 (JP); TAKEI, Masahiro, Chiba-shi, Chiba 263-8522 (JP)
(74) Representative: Santarelli
(86) International application number: PCT/JP2024/034824
(87) International publication number: WO 2025/070798

(57) **Abstract**

An electrical tomography device visualizes at least any one of the distribution of permittivity or the distribution of conductivity of a measurement object by using a complex sensitivity matrix.

## Description

### TECHNICAL FIELD

The present invention relates to an electrical tomography method and an electrical tomography device. Priority is claimed on Japanese Patent Application No. 2023-170589, filed September 29, 2023, the content of which is incorporated herein by reference.

### BACKGROUND ART

The imaging technology for cells and tissues is an important technology in a wide range of fields. In the medical field, imaging the structures and functions of cells and tissues in vivo is expected to contribute to the diagnosis of diseases and the development of therapeutic methods. In the drug discovery field, the imaging technology is applied as a technology for observing how a drug is taken up into cells and where in the cells the drug exerts its effects, in order to understand how the drug acts within the cells. Furthermore, the imaging technology for cells and tissues is also utilized to identify disease-specific targets.

Common imaging technologies for cells and tissues include fluorescence microscopy, electron microscopy, and super-resolution microscopy. Using these technologies, analyses are performed including analysis of cell morphology and function, protein localization analysis (that is, clarifying the location and amount of specific proteins and analyzing their functions and interactions within cells), cell motility analysis (that is, analyzing physiological or pathological processes by observing cell movement and morphological changes), and drug testing and drug discovery research (that is, analyzing intracellular pharmacokinetics and interactions to evaluate the efficacy and toxicity of new drugs). However, these technologies have the following problems.

First, there is a limitation in resolution. In optical microscopy, there is a limitation in resolution due to a wavelength constraint of visible light. Some of these limitations have been overcome by the development of super-resolution microscopy, but there is still a limitation in resolution.

Second, there is invasiveness. In fluorescence microscopy, it is required to label a sample with a fluorescent dye or a protein label. As a result, there is a possibility of affecting physiological processes and interactions in cells.

Third, there is a constraint on time resolution. When observing cell dynamics and physiological processes, high time resolution is required, but some technologies have a limitation in time resolution.

Due to the above-described problems and the like, it is difficult to perform imaging in a real-time and non-invasive manner on-site using the imaging technologies in the related art, and this does not lead to the use of point-of-care or the acceleration of the drug discovery process. Therefore, imaging using an electrical tomography (ET) technology may be means for solving these problems. The ET technology is used in process management and control in industrial plants and plays an important role, and in recent years, the application of the ET technology to cell observation or tissue observation in medical and drug discovery fields has been studied.

For example, as the technology that applies the ET technology to cell or tissue imaging, Non Patent Document 1 discloses the technology for imaging the concentration of cells or dead cells under cell culture using a printed circuit board. **In** addition, Non-Patent Document 2 discloses the technology for imaging the distribution of extracellular ion concentration due to ion transport in and out of cells.

### Citation List

### Non-Patent Documents

Non-Patent Document 1: Y. Yang, J. Jia, S. Smit, N. Jamil, W. Gamal, and P. O. Bagnaninchi, "A Miniature Electrical Impedance Tomography Sensor and 3-D Image Reconstruction for Cell Imaging", IEEE Sensors Journal, Vol. 17(2), 514-523, 2017.
Non-Patent Document 2: Daisuke Kawashima, Tsubasa Yuki, Songshi Li, Masahiro Takei, "Non-invasive imaging of ion concentration distribution around cell spheroids by electrical impedance tomographic sensor printed on circuit board under temporal compensation by ion transport impedance model", Biosensors and Bioelectronics, Elsevier, Vol. 212(15), 114432, 2022.

### SUMMARY OF INVENTION

### Technical Problem

The technology disclosed in Non Patent Document 1 or 2 successfully images a cell concentration or an extracellular ion concentration by measuring, using a multi-electrode sensor mounted on a printed circuit board, an impedance change caused by conductivity distribution of cells or outside the cells, and reconstructing the conductivity distribution through inverse-problem analysis.

However, in the ET technology in the related art, there is a limit to the sensitivity and accuracy, and the main cause of the limit is sensitivity matrix representation used in image reconstruction. The sensitivity matrix used in the ET technology in the related art is a sensitivity matrix created based on the conductivity, and is not generalized in dielectric spectroscopy. In dielectric spectroscopy, a phenomenon should be expressed by two physical property values of the conductivity and the permittivity in the frequency range used in the ET technology, but currently, the sensitivity matrix representation is not made in a form including the permittivity, and the reconstructed image is not an image reflecting the true phenomenon. This causes the following problems. A first problem is that permittivity cannot be reconstructed as an image; a second problem is that frequency characteristics of permittivity and conductivity cannot be reflected; and a third problem is that, as a result, it is not possible to reconstruct an image that accurately reflects dielectric properties of an object (cell constituent components such as a cell, cytoplasm, or nucleus), and therefore it is not possible to observe detailed information on cells (for example, cell composition and cellular pharmacokinetics).

The present invention has been made in view of the above-described circumstances, and an object of the present invention is to provide an electrical tomography method and an electrical tomography device capable of imaging at least any one of conductivity distribution or permittivity distribution of a measurement object by using a sensitivity matrix expressed by a complex number that has not been taken into account in the electrical tomography in the related art.

### Solution to Problem

In order to achieve the above-described object, the present invention proposes the following means.
[1] An aspect of the present invention relates to an electrical tomography method including: visualizing at least any one of the distribution of permittivity and the distribution of conductivity of a measurement object by using a complex sensitivity matrix.
[2] In the electrical tomography method according to [1], the complex sensitivity matrix may be represented by Expression (1), 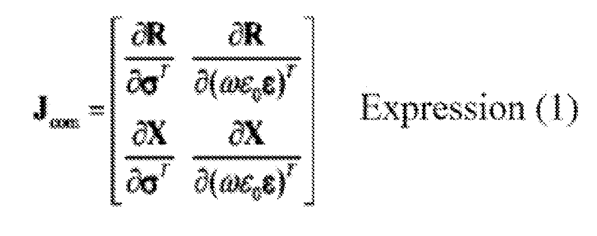 where in Expression (1),
   J_{com}: complex sensitivity matrix,
   R: resistance component (real part of complex impedance),
   X: reactance component (real part of complex impedance),
   σ: conductivity,
   ε: permittivity,
   ω: angular frequency,
   ε₀: vacuum permittivity, and
   T: transpose.
[3] In the electrical tomography method according to [2], the complex sensitivity matrix may be a complex sensitivity matrix calculated using partial derivatives of a complex sensitivity matrix based on Geselowitz theory.
[4] In the electrical tomography method according to [3], the complex sensitivity matrix may be calculated using partial derivatives of a complex sensitivity matrix derived from Expression (2) that represents a relationship between complex impedance and complex potential field obtained by modifying the Geselowitz theory, 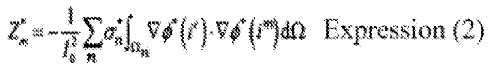 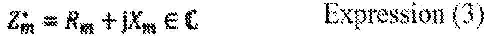 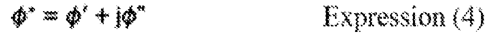 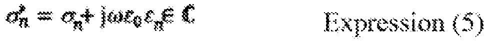 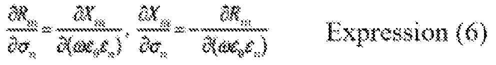 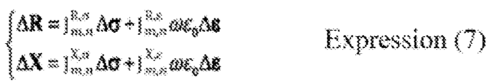 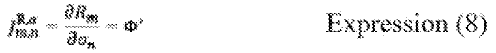 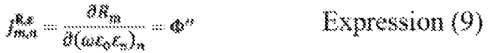 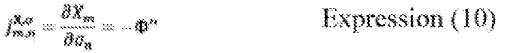 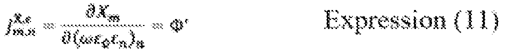 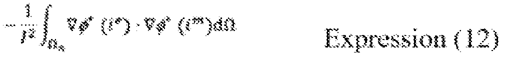 where in Expression (2),
   Zₘ*: complex impedance measured in m-th electrode pattern represented by Expression (3),
   φ*: complex potential distribution represented by Expression (4),
   σₙ*: complex conductivity of n-th mesh represented by Expression (5), and
   Ωₙ: area of n-th mesh, in Expression (3),
      Rₘ: resistance component of complex impedance measured in m-th electrode pattern, and
      Xₘ: reactance component of complex impedance measured in m-th electrode pattern, in Expression (4),
         φ': value of real part of potential, and
         φ": value of imaginary part of potential, in Expression (5),
            σₙ: conductivity of n-th mesh,
            ω: angular frequency,
            ε₀: vacuum permittivity, and
            εₙ: permittivity of n-th mesh, and
            in Expressions (3) to (5), j denotes an imaginary unit.
[5] In the electrical tomography method according to [2], the complex sensitivity matrix may be a complex sensitivity matrix calculated using partial derivatives derived from perturbations in the permittivity and the conductivity.
[6] In the electrical tomography method according to any one of [1] to [5], the distribution of at least any one of permittivity and conductivity in a plurality of dimensions of the measurement object may be visualized.
[7] In the electrical tomography method according to [6], the plurality of dimensions may be two or more dimensions selected from the group consisting of five dimensions of a frequency, a time, and a three-dimensional space.
[8] In the electrical tomography method according to any one of [1] to [7], the measurement object may be a biologically derived substance or a non-biologically derived substance.
[9] In the electrical tomography method according to [8], the biologically derived substance may be any one or more selected from the group consisting of a cell, a tissue, a living body, and a biological constituent substance.
[10] In the electrical tomography method according to [8], the non-biologically derived substance may be any one or more selected from the group consisting of liquid containing bubbles, powder, slurry, and plastic.
[11] In the electrical tomography method according to any one of [1] to [10], the measurement object may be a cell or tissue, and a concentration of ions flowing into and out of ion channels of the measurement object may be visualized by applying stimulation to the measurement object by potential control from outside the measurement object.
[12] In the electrical tomography method according to [11], a constant voltage or a periodically changing voltage may be applied to the measurement object by a single electrode or a plurality of electrodes to control movement and activity of the measurement object.
[13] Another aspect of the present invention relates to an electrical tomography device including: three or more electrodes; a calculation unit configured to calculate a complex sensitivity matrix; and a display unit configured to display at least any one of the distribution of permittivity and the distribution of conductivity of a measurement object based on the complex sensitivity matrix.
[14] In the electrical tomography device according to [13], the complex sensitivity matrix may be represented by Expression (1), 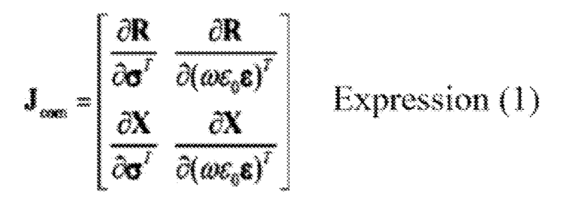 where in Expression (1),
   J_{com}: complex sensitivity matrix,
   R: resistance component (real part of complex impedance),
   X: reactance component (real part of complex impedance),
   σ: conductivity,
   ε: permittivity,
   ω: angular frequency,
   ε₀: vacuum permittivity, and
   T: transpose.
[15] **In** the electrical tomography device according to [14], the calculation unit may calculate the complex sensitivity matrix using partial derivatives of a complex sensitivity matrix based on Geselowitz theory.
[16] In the electrical tomography device according to [15], the calculation unit may derive the partial derivatives of the complex sensitivity matrix from Expression (2) that represents a relationship between complex impedance and complex potential field obtained by modifying the Geselowitz theory, 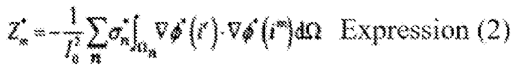 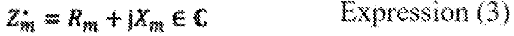 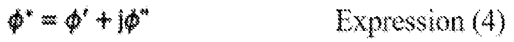 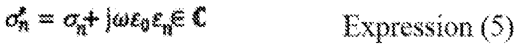 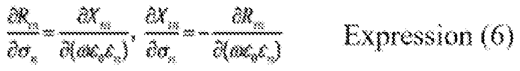 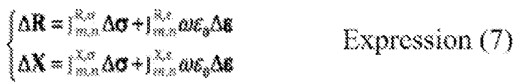 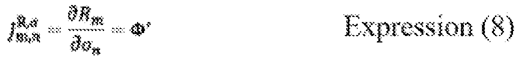 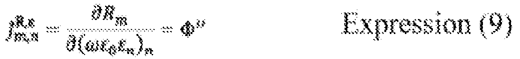 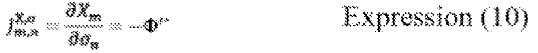 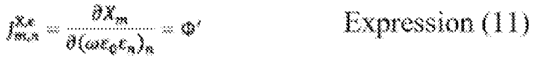 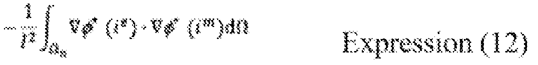 where in Expression (2),
   Zₘ*: complex impedance measured in m-th electrode pattern represented by Expression (3),
   φ*: complex potential distribution represented by Expression (4),
   σₙ*: complex conductivity of n-th mesh represented by Expression (5), and
   Ωₙ: area of n-th mesh, in Expression (3),
      Rₘ: resistance component of complex impedance measured in m-th electrode pattern, and
      Xₘ: reactance component of complex impedance measured in m-th electrode pattern, in Expression (4),
         φ': value of real part of potential, and
         φ": value of imaginary part of potential, in Expression (5),
            σₙ: conductivity of n-th mesh,
            ω: angular frequency,
            ε₀: vacuum permittivity, and
            εₙ: permittivity of n-th mesh, and
            in Expressions (3) to (5), j denotes an imaginary unit.
[17] In the electrical tomography device according to [14], the calculation unit may calculate partial derivatives derived from perturbations in the permittivity and the conductivity, and calculates the complex sensitivity matrix from the partial derivatives.
[18] In the electrical tomography device according to any one of [13] to [17], the display unit may display at least any one of the distribution of permittivity and the distribution of conductivity in a plurality of dimensions of the measurement object.
[19] In the electrical tomography device according to [18], the plurality of dimensions may be two or more dimensions selected from the group consisting of five dimensions of a frequency, a time, and a three-dimensional space.
[20] In the electrical tomography device according to any one of [13] to [19], the measurement object may be a biologically derived substance or a non-biologically derived substance.
[21] In the electrical tomography device according to [20], the biologically derived substance may be any one or more selected from the group consisting of a cell, a tissue, a living body, and a biological constituent substance.
[22] In the electrical tomography device according to [20], the non-biologically derived substance may be any one or more selected from the group consisting of liquid containing bubbles, powder, slurry, and plastic.
[23] The electrical tomography device according to any one of [13] to [22], may further include: a potential control unit configured to control potentials of the electrodes, in which the measurement object is a cell or tissue, the potential control unit applies stimulation to the measurement object by potential control from outside the measurement object, and the display unit displays the distribution of a concentration of ions flowing into and out of ion channels of the measurement object.
[24] In the electrical tomography device according to [23], the potential control unit may inject a constant voltage or a periodically changing voltage to the measurement object by a single electrode or a plurality of electrodes to control movement and activity of the measurement object.

### Advantageous Effects of Invention

According to the above-described aspect of the present invention, it is possible to image at least any one of the conductivity distribution or the permittivity distribution of the measurement object by using the sensitivity matrix expressed by the complex number that has not been taken into account in the electrical tomography in the related art.

### BRIEF DESCRIPTION OF DRAWINGS

[FIG. 1] A diagram showing the overall configuration of an electrical tomography device according to an embodiment of the present invention.
[FIG. 2] A functional block diagram of an electrical tomography device 1 according to the same embodiment.
[FIG. 3] A graph of values of J^{R,σ}_{m,n}, J^{R,ε}_{m,n}, J^{X,σ}_{m,n}, and J^{X,ε}_{m,n} when a frequency f is set to 1000 Hz, 0.1 MHz, or 1 MHz and m and n are changed.
[FIG. 4] A schematic diagram of homogeneous field simulated in an electric field simulation in Example 1.
[FIG. 5] A graph showing a change in a spatial average value <Δε> of permittivity due to a frequency for each homogeneous field in a present invention example of Example 1.
[FIG. 6] A graph showing a change in a spatial average value <Δε> of permittivity due to a frequency for each homogeneous field in a comparative example of Example 1.
[FIG. 7] (a) is a cell spheroid image by a microscope in Example 2A, (b) is an image of a cell spheroid placed on a measurement region of an electrical tomography device and electrodes arranged around the measurement region by a stereo microscope, and (c) is a contour plot showing distributions of reconstructed conductivity Δσ and reconstructed permittivity Δε at time points t₁, t₂, t₃, and t₄.
[FIG. 8] A contour plot showing distributions of reconstructed conductivity Δσ and reconstructed permittivity Δε at time points t₁, t₂, t₃, and t₄ for each frequency in Example 2B.
[FIG. 9] (a) is a microscope image of a single cell in Example 3, (b) is an image of a single cell imaged by an electrical tomography method of the present invention, and (c) is an image in which (a) and (b) are superimposed.
[FIG. 10] (a) is a diagram of a printed circuit board in an electrical tomography device used in Example 4, and (b) is a diagram showing a plurality of electrodes and a measurement region in the printed circuit board.
[FIG. 11] A graph showing a temporal change in a voltage injected to a potential control electrode and measured impedance in Example 4, in which (a) is a graph showing an impedance change under a condition in which no inhibitor is added, and (b) is a graph showing an impedance change under a condition in which an inhibitor is added.
[FIG. 12] A contour plot in which a concentration of ions is imaged based on the impedance in Example 4.
[FIG. 13] A graph showing a temporal change in conductivity for each inhibitor concentration C_{d} in hERG inhibition in Example 5.
[FIG. 14] A graph showing inhibition curves of a present invention example and a related-art example in Example 5.

### DESCRIPTION OF EMBODIMENTS

### <Electrical Tomography Device>

An electrical tomography device according to a first embodiment of the present invention will be described with reference to the accompanying drawings. In addition, dimensions and ratios of each component in the drawings do not represent actual dimensions and ratios of each component.

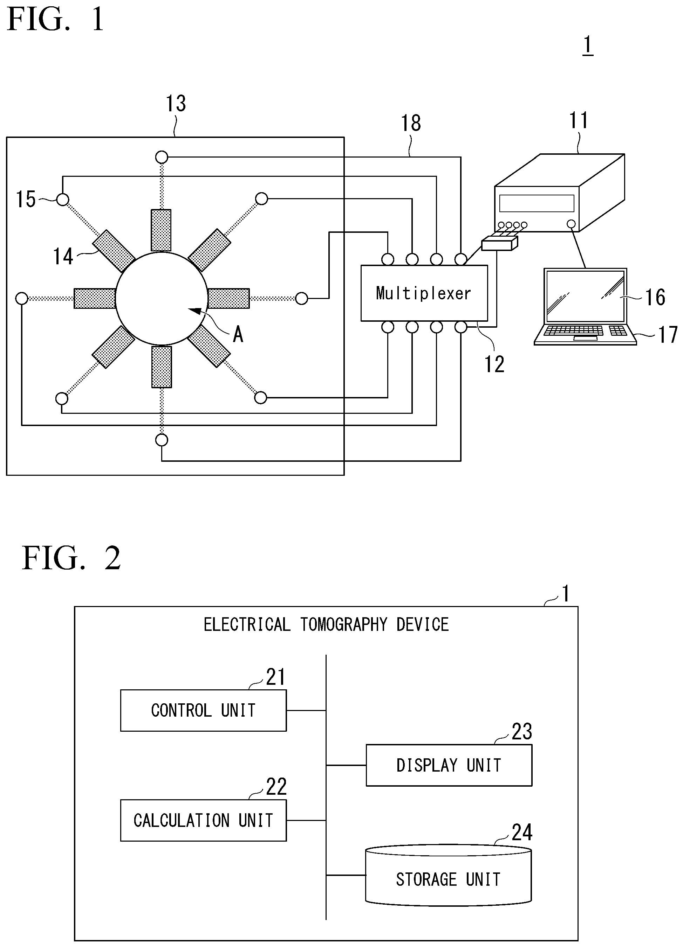

FIG. 1 is a diagram showing the overall configuration of an electrical tomography device 1 according to the present embodiment. The electrical tomography device 1 includes a current application device 11, a multiplexer 12, a printed circuit board 13, a plurality of electrodes 14, a connector 15, a display 16, a control device 17, and an electric wire 18.

The current application device 11 is a device that injects the current to the electrodes 14 arranged on the printed circuit board 13. The current application device 11 is, for example, an impedance analyzer having a function of injecting the current to the electrodes 14 by changing a frequency and an amplitude.

The multiplexer 12 is a device that switches the electrodes 14 to which the current is injected by the current application device 11 among the plurality of electrodes 14. The multiplexer 12 has a function of determining a current application and voltage measurement pattern (pattern in which two electrodes 14 are sequentially selected from the plurality of electrodes 14, the current is injected, and the voltages are sequentially measured). That is, two electrodes 14 among the electrodes 14 are selected, the current is injected, and two electrodes among the electrodes 14 are further selected to measure the voltage.

On the printed circuit board 13, the plurality of electrodes 14 and the electric wires 18 that connect the electrodes 14 and the multiplexer 12 are mounted. The electric wires 18 and the printed circuit board 13 are connected by the connector 15.

The number of electrodes 14 is, for example, 3 or more, an upper limit thereof is not particularly limited, and a high-resolution image can be displayed by the display 16 as the number of electrodes 14 increases. The number of electrodes 14 is preferably 8 or more, and more preferably 16 or more. The number of electrodes 14 may be 32 or less, or 16 or less.

The arrangement positions of the electrodes 14 are not particularly limited as long as the electrodes 14 are arranged around the measurement object, and may be determined in accordance with the shape of the measurement object; however, in order to suppress variations in measurement voltage, the electrodes 14 are preferably arranged at equal intervals. However, in the electrodes 14, some of the electrodes 14 may be arranged unevenly around the measurement object, and the interval between the electrodes 14 may be equal or unequal. The unevenness or inequality of the electrodes 14 is corrected in the calculation of a sensitivity matrix (complex sensitivity matrix) expressed by a complex number. In FIG. 1, eight electrodes are arranged at equal intervals around a measurement region A.

Furthermore, the electrodes 14 can also be used for potential control of cells or tissues. In this case, the electrodes 14 are arranged, for example, in the measurement region A. The electrodes 14 can image at least any one of the permittivity and the conductivity while the ion channel activity is controlled.

The display 16 images at least any one of the distribution of the permittivity or the distribution of the conductivity of the measurement object. The display 16 may be a known display, but preferably has sufficient resolution corresponding to the number of meshes.

The control device 17 controls various operations of the electrical tomography device 1. The control device 17 includes, for example, a central processing unit (CPU) and a storage device such as a read-only memory (ROM), a random-access memory (RAM), or a hard disk drive (HDD)/solid-state drive (SSD). The control device 17 is implemented, for example, by executing a predetermined program in the CPU. The program may be acquired via a recording medium or via a network. In addition, a dedicated hardware configuration for implementing the configuration of the electrical tomography device 1 may be used. Hereinafter, each of the configurations will be described.

FIG. 2 is a functional block diagram of the electrical tomography device 1 according to the present embodiment. As shown in FIG. 2, the electrical tomography device 1 includes a control unit 21, a calculation unit 22, a display unit 23, and a storage unit 24.

The control unit 21 has a function of controlling an overall operation of the electrical tomography device 1. The control unit 21 is implemented, for example, by causing the CPU provided in the control device 17 to execute a program.

The calculation unit 22 calculates the complex sensitivity matrix. The calculation of the complex sensitivity matrix will be described in detail in the description of an electrical tomography method.

The display unit 23 displays at least any one of the distribution of the permittivity or the distribution of the conductivity of the measurement object based on the complex sensitivity matrix. The display unit 23 is implemented by, for example, the display 16.

The storage unit 24 stores information required for the calculation performed by the calculation unit 22, information related to a calculation process, and a calculation result. Further, the storage unit 24 stores a program for the control unit 21 to perform various types of control. The storage unit 24 is implemented by a ROM, a RAM, an HDD, an SSD, or the like.

### <Electrical Tomography Method>

Hereinafter, an electrical tomography method according to a second embodiment of the present invention will be described. The electrical tomography method is an electrical tomography method of visualizing at least any one of the distribution of the permittivity or the distribution of the conductivity of the measurement object by using the complex sensitivity matrix. The complex sensitivity matrix is a sensitivity matrix indicating how much the measurement voltage changes when the current is injected with respect to a change in electrical physical properties (conductivity and permittivity) distributed in a space, and has an imaginary component. The complex sensitivity matrix is represented by the permittivity, the conductivity, and a real part and an imaginary part of the complex impedance. The complex sensitivity matrix varies depending on a spatial distribution of the electrical physical properties of the measurement object, and the like, and the electrical physical property distribution can be calculated when the complex sensitivity matrix is known. The complex sensitivity matrix is calculated based on a predetermined current application and voltage measurement pattern, mesh coordinates, and coordinates of the plurality of electrodes.

The current application and voltage measurement pattern refers to a pattern in which two electrodes are sequentially selected from the plurality of (three or more) electrodes, the current is injected, and a potential difference between the electrodes is measured, and the number of current application and voltage measurement patterns varies depending on a method of measuring the potential difference, such as an adjacent method, a reference method, or a two-terminal method. Hereinafter, the total number of current application and voltage measurement patterns is denoted by M. Further, hereinafter, an m-th current application and voltage measurement pattern may be described, and, in this case, m is an integer of 1 or more and M or less.

Next, a method of determining the mesh coordinates will be described. For example, a region (measurement region) in which the measurement object is arranged is divided into a two-dimensional mesh in accordance with the number of electrodes such that an appropriate resolution is obtained. For example, when the number of electrodes is eight, the measurement region can be divided into 4096 points in total by dividing the measurement region into 64 parts in the x direction and approximately 64 parts in the y direction to create a mesh. The mesh refers to each divided region, and the coordinates of each mesh refer to the mesh coordinates. In the above-described example, the number of meshes N is 4096. Hereinafter, the n-th mesh among the plurality of meshes may be described, and, in this case, n is an integer of 1 or more and N (the number of meshes) or less.

Hereinafter, the electrical tomography method of visualizing (displaying) the distribution of the conductivity and the distribution of the permittivity in a total of five dimensions of a frequency, a time, and a three-dimensional space will be described with an example of the complex sensitivity matrix. In addition, the frequency is a frequency of an alternating current flowing from the electrodes.

A linear expression for an inverse problem of five-dimensional electrical tomography is represented by Expression (1), similarly to electrical impedance tomography (EIT) in the related art.

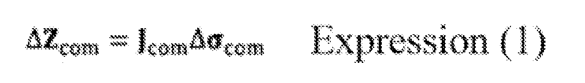

In Expression (1), Z_{com} denotes a column vector of complex impedance in a plurality of current application and voltage measurement patterns, J_{com} denotes a complex sensitivity matrix, and σ_{com} denotes a column vector of the distribution of complex conductivity. Here, Δ denotes a difference from a reference value. As the reference value, a value for the measurement region A in a state in which the measurement object is not arranged is used. For example, when the measurement object is a cell, a value in a state in which only a culture medium is arranged in the measurement region A is used as the reference value. In Expression (1), ΔZ_{com} is represented by Expression (2), and Δσ_{com} is defined by Expression (3).

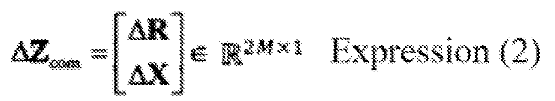

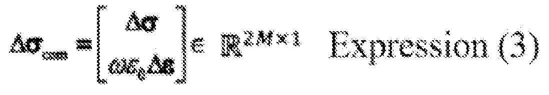

In Expression (2), ΔR denotes a column vector of a difference from a reference value of resistance, and ΔX denotes a column vector of a difference from a reference value of reactance. In Expression (3), Δσ denotes a column vector of a difference from a reference value of conductivity, Δε denotes a column vector of a difference from a reference value of relative permittivity, ω is an angular frequency, and ε₀ denotes vacuum permittivity. In addition, M in Expression (2) denotes the total number of current application and voltage measurement patterns, and N in Expression (3) denotes the total number of meshes in which the distribution of the conductivity is discretized. The sizes of the matrices of ΔR, ΔX, Δσ, and Δε are defined as in Expressions (4), (5), (6), and (7), respectively.$ΔR ∈ {ℝ}^{M \times 1}$ Expression (4) $ΔX ∈ {ℝ}^{M \times 1}$ Expression (5) $Δσ ∈ {ℝ}^{N \times 1}$ Expression (6) $Δ ε ∈ {ℝ}^{N \times 1}$ Expression (7)

Therefore, the sizes of the matrices of Z_{com}, σ_{com}, and the complex sensitivity matrix J_{com} are defined as in Expressions (8), (9), and (10), respectively.

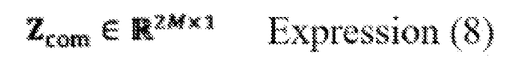

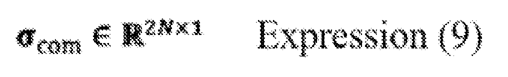

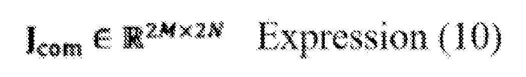

From Expressions (1) to (3), the complex sensitivity matrix J_{com} is represented by four components as in Expression (11).

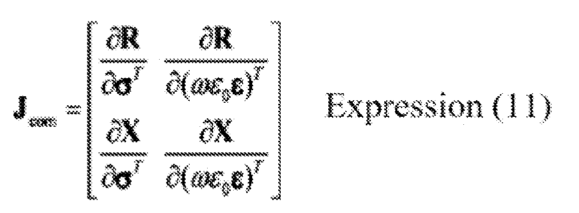

In Expression (11), J_{com} denotes a complex sensitivity matrix, R denotes a resistance component (real part of complex impedance), X denotes a reactance component (real part of complex impedance), σ denotes conductivity, ε denotes permittivity, ω denotes an angular frequency, ε₀ denotes vacuum permittivity, and T denotes a transpose. Expression (12) is obtained from Expressions (1) to (3) and (11).

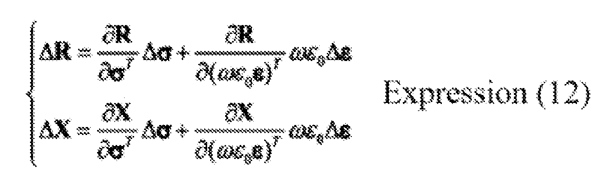

A formula of the sensitivity matrix based on the Geselowitz theory can be applied to derive each component of the complex sensitivity matrix J_{com}. By modifying the Geselowitz theory, a relationship between the complex impedance and the complex potential field is represented by Expression (13).

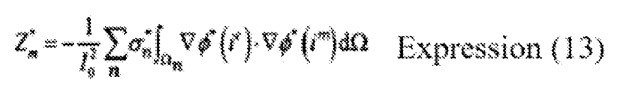

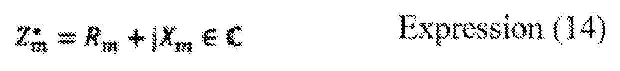

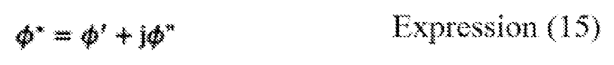

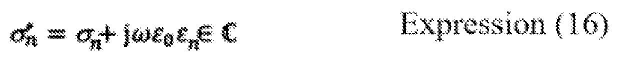

In Expression (13), Z_{m*}: denotes complex impedance measured in the m-th current application and voltage measurement pattern, and is represented by Expression (14). In Expression (14), Rₘ denotes a resistance component of the complex impedance measured in the m-th electrode pattern, and Xₘ denotes a reactance component of the complex impedance measured in the m-th electrode pattern.

In Expression (13), φ* denotes a complex potential distribution represented by Expression (15), φ*(i^{e}) denotes a complex electric potential field generated by causing the current i to flow through the e-th electrode, and φ*(i^{m}) denotes a complex electric potential field generated by causing the current i to flow through the m-th electrode. In Expression (15), φ' denotes a value of a real part of a potential, and φ" denotes a value of an imaginary part of a potential.

In Expression (13), σₙ* denotes complex conductivity of an n-th mesh, and is represented by Expression (16). In Expression (16), σₙ denotes conductivity of the n-th mesh, ω denotes an angular frequency, ε₀ denotes vacuum permittivity, and εₙ denotes permittivity of the n-th mesh.

In Expression (13), Ωₙ denotes an area of the n-th mesh. In Expressions (14) to (16), j denotes an imaginary unit. In addition, since an injected current I₀ in Expression (13) is a constant value in all measurements, I₀² in Expression (13) is ignored in the calculation of the sensitivity matrix. Therefore, by deriving four partial derivatives from Expression (13), the complex sensitivity matrix J_{com} in Expression (11) is obtained. These four partial derivatives satisfy the Cauchy-Riemann relationship, that is, the relationship of the four components of the complex sensitivity matrix J_{com} is represented as in Expression (17).

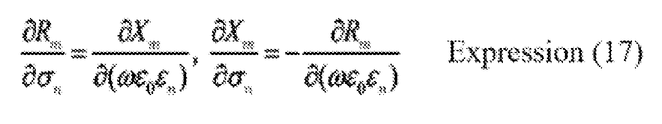

As described above, Expression (12) is rewritten as in Expression (18). In Expression (18), J^{R,σ}_{m,n}, j^{R,ε}_{m,n}, J^{X,σ}_{m,n}, and J^{X,ε}_{m,n} are represented by Expressions (19), (20), (21), and (22), respectively. In addition, φ' in Expression (19) and Expression (22) denotes a real part on the left side of Expression (23), and φ" in Expression (20) and Expression (21) denotes an imaginary part on the left side of Expression (23).

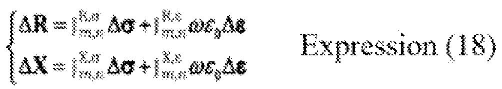

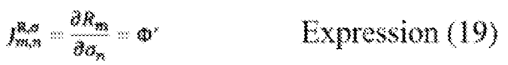

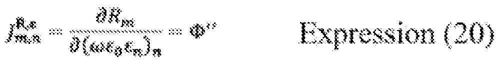

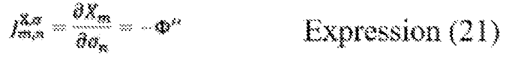

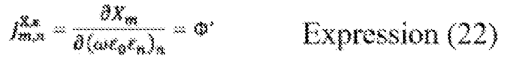

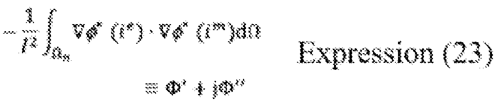

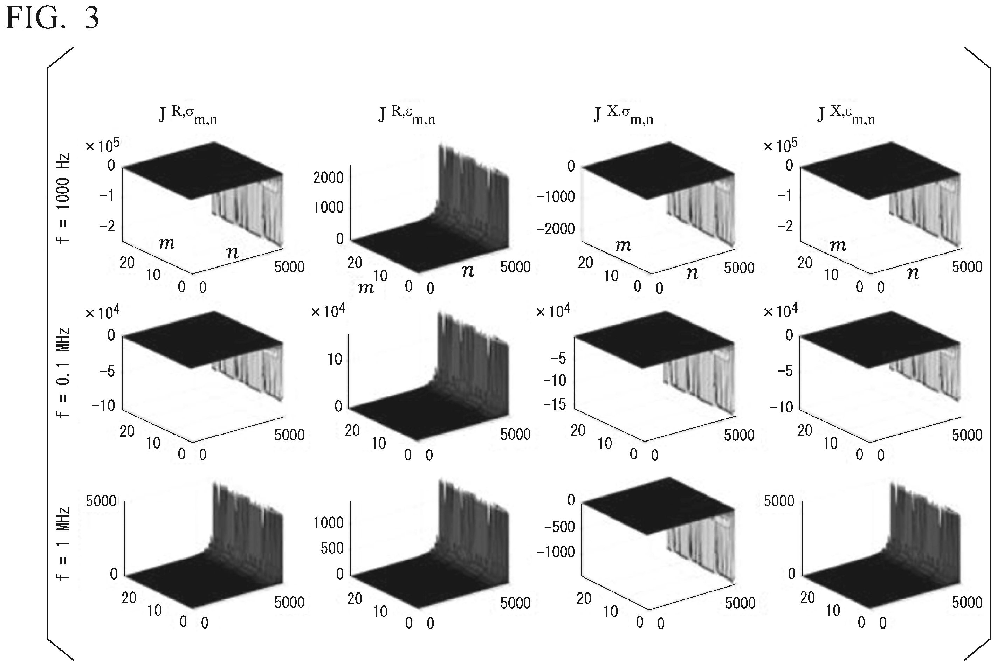

The complex conductivity is imaged by an inverse problem of Expression (18). For example, the complex conductivity is imaged by image reconstruction using numerical optimization by Tikhonov regularization or Gauss-Newton. The method of imaging the complex conductivity is not limited to this. The real part of the complex conductivity represents the conductivity, and the imaginary part of the complex conductivity represents the permittivity. FIG. 3 shows graphs of values of J^{R,σ}_{m,n}, J^{R,ε}_{m,n}, J^{X,σ}_{m,n}, and J^{X,ε}_{m,n} when the frequency f is set to 1000 Hz, 0.1 MHz, or 1 MHz and m and n are changed. J^{R,σ}_{m,n}, J^{R,ε}_{m,n}, J^{X,σ}_{m,n}, and J^{X,ε}_{m,n} are changed depending on the frequency, and it is shown that the complex sensitivity matrix J_{com} is a function of the frequency.

In the present embodiment, a biologically derived substance or a non-biologically derived substance can be used as targets.

The biologically derived substance is, for example, one or more selected from the group consisting of a cell, a tissue, a living body, and a biological constituent substance. Examples of the biological constituent substance include ions, proteins, organelles, and cell nuclei.

The non-biologically derived substance is not a substance collected from a living body, but is generated, for example, in a process of manufacturing an industrial product. Examples of the non-biologically derived substance include liquid containing air bubbles, powder, slurry, and plastic. Therefore, the non-biologically derived substance may be one or more selected from the group consisting of liquid containing air bubbles, powder, slurry, and plastic.

In the electrical tomography method according to the present embodiment, the object is a cell or tissue, and by applying stimulation to the object from the outside of the object by potential control, it is possible to visualize the concentration of ions flowing in and out of the ion channel of the object. Also, the stimulation may be performed by steady-state values or periodic potential control of a plurality of electrodes. Therefore, in the electrical tomography method according to the present embodiment, a constant voltage or a periodically changing voltage may be injected to the measurement object by a single electrode or a plurality of electrodes to control the movement and the activity of the measurement object. Further, the electrical tomography device according to one embodiment of the present invention may include a potential control unit that controls a potential of the electrode. The potential control unit may inject a constant voltage or a periodically changing voltage to the measurement object by a single electrode or a plurality of electrodes to control the movement and the activity of the measurement object.

It is found that, with the above-described electrical tomography method and electrical tomography device, it is possible to observe various structures and dynamics in cells, and it is possible to clarify phenomena that are difficult to observe with the related-art methods, such as the activity of ion channels in cells and the distribution of intracellular fluid. Further, since this technology is non-invasive, it is possible to observe the cells while maintaining the physiological state of the cells. The application of such electrical tomography has various possibilities in the fields of life science and medicine, and, for example, it is possible to analyze in detail the pathological changes of cells, the drug response, and the like. Further, it is also useful for observing electrical activity of nerve cells, cardiomyocytes, and the like.

The present invention has been described above with reference to the present embodiment. However, the technical scope of the present invention is not limited to the embodiment described above, and various modifications can be made without departing from the gist of the present invention.

For example, in the embodiment described above, the complex sensitivity matrix is calculated by the partial derivatives of the complex sensitivity matrix based on the Geselowitz theory, but the complex sensitivity matrix may be calculated by the partial derivatives derived from the perturbation of the permittivity and the conductivity.

In addition, in the embodiment described above, the distribution of the conductivity and the distribution of the permittivity are visualized in a total of five dimensions of the frequency, the time, and the three-dimensional space, but the dimensions for visualization are not limited to this. Therefore, the electrical tomography method according to the present disclosure may visualize the distribution of at least any one of the permittivity and the conductivity in the plurality of dimensions of the object.

In addition, the distribution of the conductivity and the distribution of the permittivity may be visualized in two or more dimensions of the five dimensions of the frequency, the time, and the three-dimensional space. In other words, the above-described plurality of dimensions may be two or more dimensions selected from the group consisting of the five dimensions of the frequency, the time, and the three-dimensional space.

In addition, in the embodiment described above, the four partial derivatives of the complex sensitivity matrix are derived based on the Geselowitz theory, but the present invention is not limited to this, and the derivation can also be performed by another method. For example, the present invention can also be applied to a general sensitivity matrix derivation method such as a method using the perturbations (ΔR, ΔX) of the complex impedance due to the perturbations (Δσ, Δε) of the conductivity and the permittivity.

In addition, in the embodiment described above, a case has been described in which the current is injected to the electrode to measure the voltage, but electrical tomography may be performed by injecting the voltage to the electrode to measure the current. In such a case, the electrical tomography device includes a voltage application device instead of the current application device.

The electrical tomography device can be applied not only to a plurality of concentric electrode arrays as shown in FIG. 1, but also to a linear electrode array or a two-dimensional or three-dimensional multilayered electrode array. By devising the electrode array, it is possible to image the frequency and the two-dimensional and three-dimensional spatiotemporal distributions.

In addition, a glass substrate, a rigid printed board, or a flexible printed board can be applied to the substrate on which the electrode is mounted. The electrode may be mounted on a wearable material, a pipe, a chamber, or the like, and can be designed depending on the application.

### Examples

The effects of one aspect of the present invention will be described in more detail with reference to Examples. However, a condition in the Examples is merely one condition example adopted to confirm the feasibility and effects of the present invention. The present invention is not limited to the one condition example. The present invention may adopt various conditions without departing from the gist of the present invention, as long as the objects of the present invention are achieved.

### (Example 1)

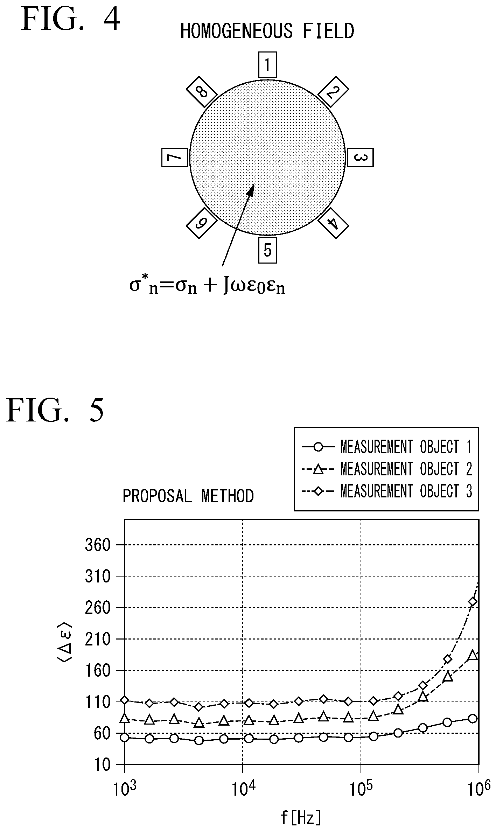

In the fields (homogeneous fields) 1 to 3 (measurement objects 1 to 3 in Table 1) having circular homogeneous conductivity and permittivity as shown in FIG. 4, a case where the permittivity of the field was uniformly changed was assumed, and the spatial average value <Δε> of the permittivity reconstructed by the electrical tomography method according to the present invention and an electrical impedance tomography method in the related art was obtained by electric field simulation. In the present simulation, eight electrodes were arranged at equal intervals along the outer periphery of the homogeneous field. The frequency range was set to 1 kHz to 1 MHz. The conductivity σₙ and the permittivity εₙ of the homogeneous fields (measurement objects) 1 to 3 and the reference values thereof are shown in Table 1.

**[Table 1]**

| Reference value | σₙ | εₙ |
|---|---|---|
| | 0.0010 | 100 |

| Measurement object | **σₙ** | εₙ |
|---|---|---|
| 1 | 0.0010 | 150 |
| 2 | 0.0010 | 200 |
| 3 | 0.0010 | 500 |

### [Present Invention Example]

In the electrical tomography method of the present invention example, the permittivity change Δε was calculated by the inverse problem of Expression (18) using the complex sensitivity matrix of Expression (19) to Expression (22) obtained as the partial derivatives of Expression (13). The results are shown in FIG. 5. FIG. 5 is a graph showing the change in the spatial average value <Δε> of the permittivity due to the frequency for each homogeneous field in the present invention example.

### [Comparative Example]

The following method was adopted in the electrical impedance tomography method in the related art. That is, the imaginary part (reactance ΔX) of the impedance and the imaginary part (permittivity) of the complex conductivity were obtained based on Expression (24) described by the Jacobian matrix J in the related art instead of ΔZ_{com} and J_{com} of Expression (1).$ΔX = J \left({ωε}_{0}Δε\right)$ Expression (24)

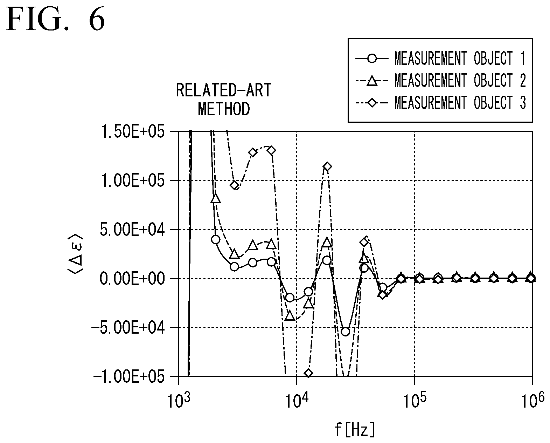

The results are shown in FIG. 6. FIG. 6 is a graph showing the change in the spatial average value <Δε> of the permittivity due to the frequency for each homogeneous field in the comparative example.

As shown in FIGS. 5 and 6, in the electrical impedance tomography method in the related art, the reconstructed permittivity varied for each frequency, and unstable permittivity was exhibited. On the other hand, in the electrical tomography method according to the present invention example, stable permittivity was reconstructed at any frequency. In the related art method of the comparative example, it was presumed that the theoretical correlation between the real part (resistance) and the imaginary part (reactance) of the impedance and the complex conductivity was not taken into account, and the behavior of the reactance that changes significantly depending on the frequency was reflected in the reconstructed permittivity, resulting in unstable behavior.

### (Example 2A)

A cell spheroid was used as the measurement object, and the conductivity and the permittivity of the cell spheroid were imaged. The experimental device was configured with an impedance analyzer, a multiplexer, a sensor, and a control PC. A printed circuit board was used as the sensor, and eight electrodes were provided on the board at equal intervals on a concentric circle. The impedance between each electrode pair (a total of 28 patterns) was measured by selecting and switching two electrodes among the eight electrodes using the multiplexer. For the experiment, a cell spheroid formed on a well plate was used, which was derived from human fetal lung fibroblasts MRC-5. As the experimental protocol, first, the cell spheroid was arranged in the center portion of the sensor, and then a deionized sucrose solution with adjusted osmotic pressure was added dropwise around the cell spheroid. In this way, the system in which ions are transported from the cell spheroid to the surrounding sucrose solution was formed. In this case, the impedance measurement between each electrode pair was started, and the time-dependent impedance change associated with the ion transport that occurred around the cell spheroid was acquired. In Example 2A, ωε₀ was not taken into account. Stated another way, ωε₀ in the complex sensitivity matrix represented by Expression (11) was set to 1. In addition, the four partial derivatives in the complex sensitivity matrix represented by Expression (11) were obtained from R and X at the frequency of 1 kHz without considering the frequency change.

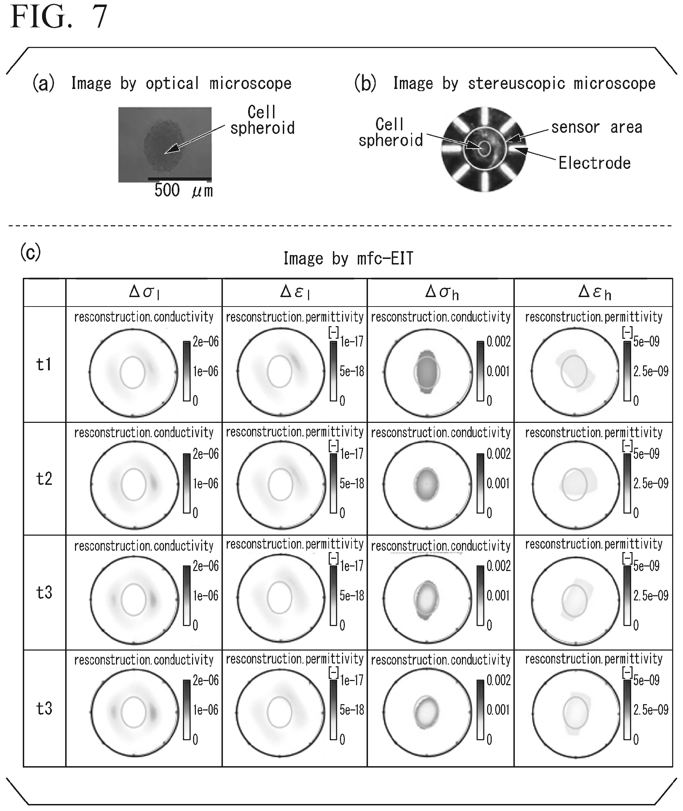

(a) of FIG. 7 is a cell spheroid image taken by a microscope, and is a reference image of the cell spheroid shape or the diameter. (b) of FIG. 7 is an image of the cell spheroid placed on the measurement region of the electrical tomography device and the electrodes arranged around the measurement region, taken by a stereo microscope. In (b) of FIG. 7, the inside of the circle marked with sensor area indicates a region in which the sensor is arranged, the circle marked with Cell spheroid indicates a boundary of the cell spheroid, and the inside of the circle is a region in which the cell spheroid is shown. (c) of FIG. 7 is a contour plot showing the distributions of the reconstructed conductivity Δσ and the reconstructed permittivity Δε at time points t₁, t₂, t₃, and t₄. Time elapses in the order of t₁ to t₄. In (c) of FIG. 7, Δσ₁ and Δε₁ are images of the conductivity distribution and the permittivity distribution at a low frequency band (1 kHz), respectively, and Δσₕ and Δεₕ, are images of the conductivity distribution and the permittivity distribution at a high frequency band (1 MHz), respectively. In addition, the ellipse arranged inside in (c) of FIG. 7 corresponds to the circle marked with Cell spheroid in (b) of FIG. 7.

Each contour plot of Δσ₁ in (c) of FIG. 7 shows the extracellular conductivity distribution. It was shown that the extracellular conductivity was increased with the elapse of time. The increase in the conductivity was due to the increase in the extracellular ionic concentration caused by the movement of ions from the intracellular space to the extracellular space. In addition, the intracellular conductivity was almost zero because the low-frequency current could not pass through the cell membrane.

As shown in each contour plot of Δε₁, the distribution of the permittivity in the extracellular space could be shown. This was because the extracellular ionic concentration was increased due to the ion movement caused by the extracellular solution, and thus the extracellular permittivity was decreased over time.

As shown in each contour plot of Δσₕ, the distribution of the intracellular conductivity is shown. The distribution of the intracellular conductivity was due to the fact that the high-frequency current could pass through the cell membrane and that many free ions were present in the intracellular space. The intracellular conductivity was decreased with the elapse of time because the ions were moved to the outside.

As shown in each contour plot of Δεₕ, the permittivity distribution in the cell could be shown, and it was shown that the permittivity distribution decreased with the elapse of time.

As described above, with the electrical tomography method according to the embodiment of the present invention, it was possible to image the distribution of the conductivity and the distribution of the permittivity in five dimensions of the frequency, the time, and the space.

### (Example 2B)

Using the experimental device and the experimental protocol in the same manner as in Example 2A, temporal changes in impedance associated with ion transport that occurred around the cell spheroid were acquired. In the present Example 2B, based on the acquired impedance of 28 patterns, the time-space and frequency distributions of the conductivity and the permittivity inside and outside the cell spheroid were reconstructed by considering ωε₀ and further using the complex sensitivity matrix represented by Expression (11) as the function of the frequency (or each frequency ω) of R and X.

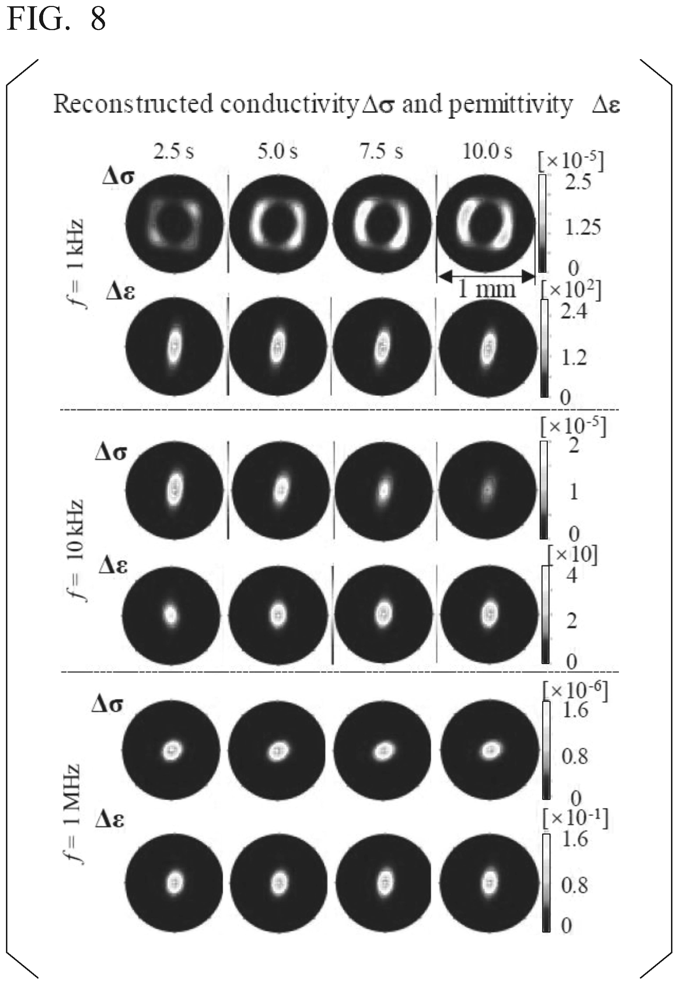

FIG. 8 is a contour plot showing the distributions of the reconstructed conductivity Δσ and the reconstructed permittivity Δε at each frequency (f = 1 kHz, 10 kHz, and 1 MHz) at time points t₁ = 2.5 s, t₂ = 5.0 s, t₃ = 7.5 s, and t₄ = 10.0 s in Example 2B.

By using the complex sensitivity matrix represented by Expression (11) and treating ωε₀, R, and X as frequency functions, it was possible to reconstruct more accurate and more stable images of the distribution of the permittivity and the time-space distribution and the frequency distribution of the conductivity of the measurement object as shown in FIG. 8.

### (Example 3)

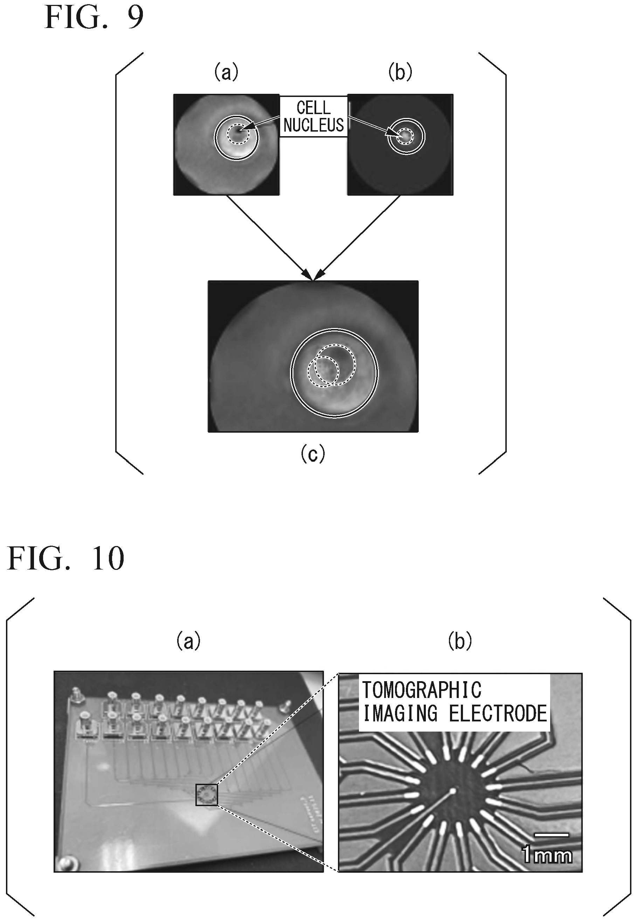

A single cell was imaged by the electrical tomography method according to the present disclosure. Specifically, the change in the conductivity inside the cell was measured by using a plurality of electrodes with the frequency of the current in the MHz band. In this measurement, a minute electrode array was arranged around the cell. A weak current flowed into the cell by adjusting the frequency range, and the image reconstruction was performed based on the measured impedance. (a) of FIG. 9 is a microscope image of a single cell, (b) of FIG. 9 is an image of a single cell imaged by the electrical tomography method according to the present disclosure, and (c) of FIG. 9 is an image in which (a) and (b) of FIG. 9 are superimposed. In these images, the region inside the solid line indicates the single cell, and the region inside the dashed line indicates the cell nucleus of the single cell. As shown in (a) to (c) of FIG. 9, it was shown that a high-resolution single cell image could be obtained and the internal structure of the single cell could be imaged.

### (Example 4)

Targeting a voltage-dependent potassium channel (hERG), which is an ion channel present in cardiomyocytes, changes in impedance values and extracellular ion concentrations were imaged, as an evaluation of hERG inhibition by drug action, under conditions with and without addition of the hERG inhibitor. (a) of FIG. 10 shows a printed circuit board in the electrical tomography device used in the present example, and (b) of FIG. 10 is a diagram showing a plurality of electrodes and a measurement region in the printed circuit board. As shown in (b) of FIG. 10, 16 electrodes were arranged in the measurement region in a plane of the printed circuit board at equal intervals on a concentric circle. In addition, the potential control electrode was provided at the center of the measurement region. A mechanism was provided in which the membrane potential required for channel activity could be controlled by injecting the pulse potential to the potential control electrode. The experimental device was configured with a sensor, an impedance analyzer (IM3570, manufactured by HIOKI E.E. CORPORATION), a multiplexer, a function generator, and a control and data integration PC. The current application and the voltage measurement were repeated over time with the plurality of electrode pairs, and the temporal change in the impedance value in the plurality of electrode pairs was acquired. Further, after a certain time elapsed after the start of the measurement, the pulse signal output from the function generator was applied to the potential control electrode to control the cell membrane potential. HEK293 cells expressing hERG (human embryonic kidney cells 293) were used as the cells. A rectangular pulse wave of -70 mV was applied to the potential control electrode in HEK293 seeded on the sensor to activate hERG activity. In addition, as the hERG inhibition experiment, astemizole, which is well known as an hERG inhibitor, was dissolved in a dimethyl sulfoxide (DMSO) medium and mixed in advance with the HEK293 cell suspension, and measurement was performed by the same method as described above.

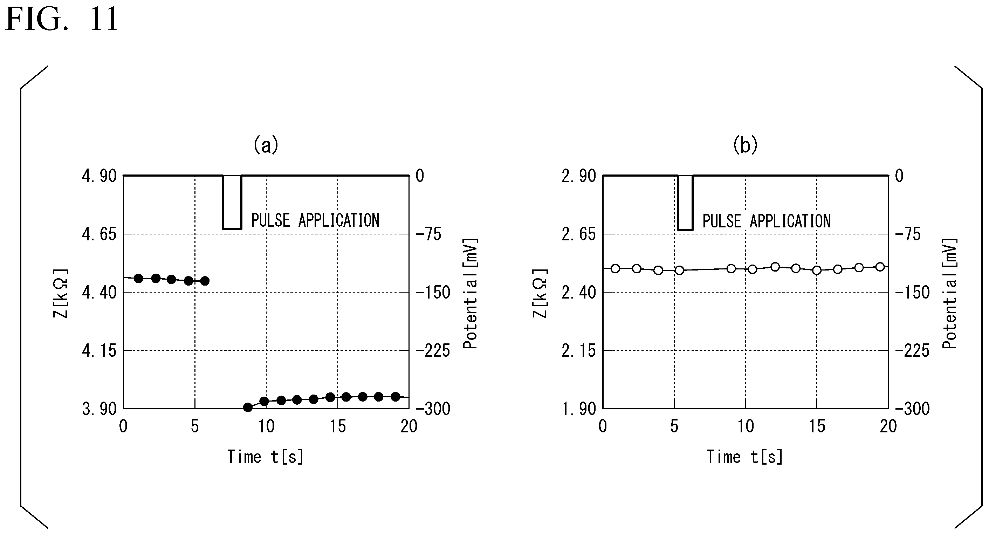

FIG. 11 shows the temporal change in the voltage injected to the potential control electrode and the measured impedance. (a) of FIG. 11 shows the impedance change under the condition without the inhibitor, and it was confirmed that the impedance was rapidly decreased after the application of the rectangular pulse wave of - 70 mV. This was because the hERG activity was generated by the potential application, and the potassium ions were released to the extracellular space, resulting in the increase in the ion concentration in the extracellular fluid.

In addition, (b) of FIG. 11 shows the impedance change under the condition in which the inhibitor was added. Under the condition in which the inhibitor was mixed, the impedance changes before and after the pulse potential application were relatively small, and the hERG inhibition by the addition of astemizole was captured.

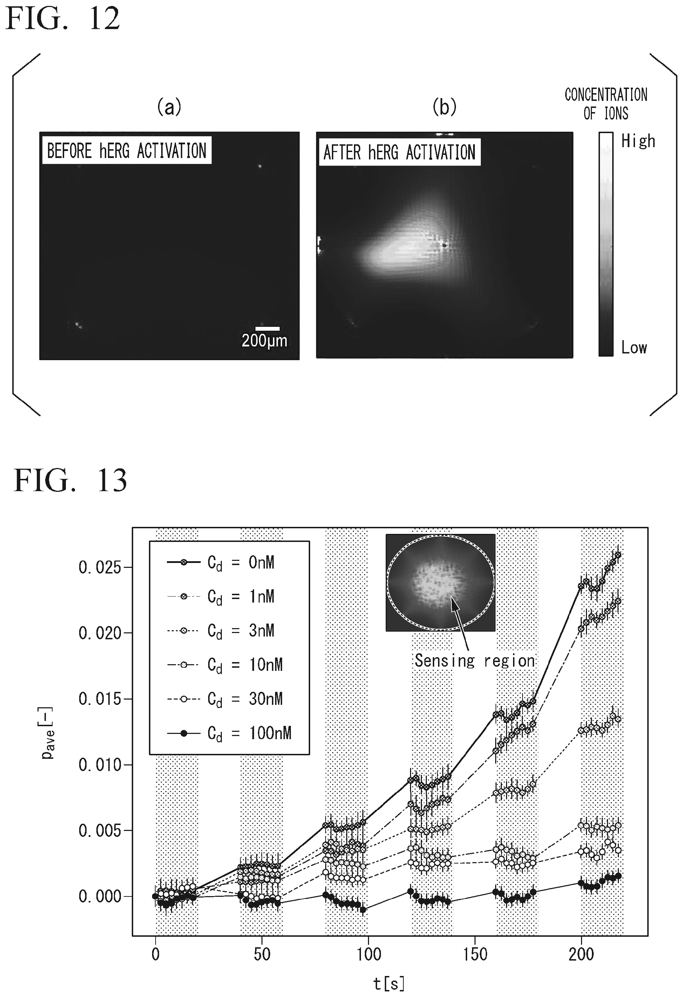

Next, FIG. 12 shows the results of performing tomography of the concentration of ions based on the impedance data obtained in a plurality of current application and voltage measurement patterns. As shown in FIG. 12, a state in which the extracellular ion concentration changed before and after the activity of hERG could be imaged.

### (Example 5)

The temporal change in the extracellular ion concentration was obtained from the imaging of the extracellular ion concentration, and the toxicity evaluation of the inhibitor was performed by drawing the inhibition curve showing the relationship between the inhibitor concentration and the ion channel activity. The hERG inhibitors having different concentrations were added, and the temporal change in the conductivity was measured. In the present example, the same electrical tomography device as that in Example 4 was used to acquire the temporal change in the conductivity. The same cells as the cells in Example 4 were used, E-4031 was used as the hERG inhibitor, and the hERG inhibitor was mixed in advance in the cell suspension such that the inhibitor concentration c_{d} was different.

In addition, the present invention example and the related-art example were compared using the inhibition curve according to the patch-clamp method disclosed in Z. Zhou et al., Properties of HERG Channels Stably Expressed in HEK 293 Cells Studied at Physiological Temperature, Biophysical Journal, 74,1998, 230-241., as the related-art example.

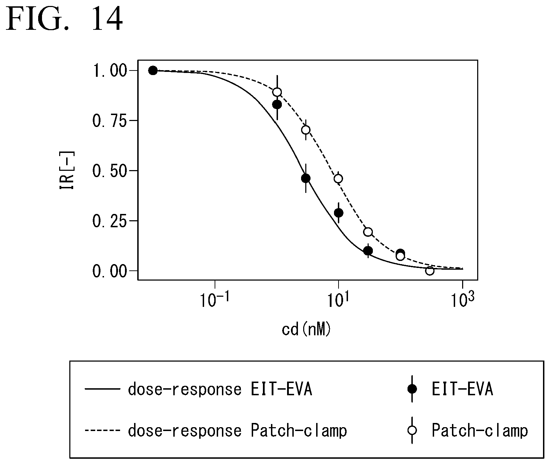

FIG. 13 is a graph showing the temporal change in the conductivity for each inhibitor concentration C_{d}. FIG. 14 is a graph showing the inhibition curves of the present invention example and the related-art example. In FIG. 13, a white region in which the plot is not shown indicates the time during which the voltage is injected. In the present invention example, the inhibition curve of FIG. 14 was created using the conductivity measured after the fifth voltage application from the start of measurement (after 200 seconds from the start of measurement). In FIG. 14, the curve indicated by the solid line is the inhibition curve of the present invention example, and the curve indicated by the dashed line is the inhibition curve of the related-art example.

In the present invention example, for example, the inhibitor concentration c_{d} when the activity was 50% (IR = 0.50) was calculated to be 2 nM, whereas the inhibitor concentration c_{d} when the activity was 50% in the related-art example was calculated to be 7 nM. This degree of difference is generally acceptable. Therefore, it was found that, in the present invention example, the 50% inhibitory concentration IC50 could be evaluated non-invasively while maintaining the physiological state of the cells. Furthermore, since the evaluation could be performed more easily than in the related art, the operator could perform accurate processing with fewer errors.

The electrical tomography device and the electrical tomography method according to the present disclosure have been described above. The permittivity can be reconstructed into an image by the electrical tomography device and the electrical tomography method according to the present disclosure. In addition, the electrical tomography device and the electrical tomography method according to the present disclosure can reflect the frequency characteristics of the permittivity and the conductivity. Furthermore, since the electrical tomography device and the electrical tomography method according to the present disclosure can reconstruct an image that accurately reflects dielectric properties of cell constituent components such as cells, cytoplasm, and nuclei, it is possible to observe detailed information on cells (for example, cell composition and cellular pharmacokinetics). Specifically, for example, at least any one of the conductivity distribution and the permittivity distribution of the biologically derived substance or the non-biologically derived substance can be imaged in five dimensions. As a result, it is possible to rapidly and in real time obtain, for example, analyses of pharmacokinetics for cultured cells, induced pluripotent stem cells, membrane proteins such as ion channels, and living tissues, and detection of cancer cells, thereby enabling provision of an innovative imaging technology for the medical field and the drug discovery field. The present disclosure is also applicable to process management and control in, for example, chemical plants, and contributes to the SDGs, including energy-related issues.

### REFERENCE SIGNS LIST

1 Electrical tomography device
11 Current application device
12 Multiplexer
13 Printed circuit board
14 Electrode
15 Connector
16 Display
17 Control device
18 Electric wire
21 Control unit
22 Calculation unit
23 Display unit
24 Storage unit

## Claims

1. An electrical tomography method comprising:
visualizing at least any one of a distribution of permittivity and a distribution of conductivity of a measurement object by using a complex sensitivity matrix.

2. The electrical tomography method according to Claim 1,
wherein the complex sensitivity matrix is represented by Expression (1),
where in Expression (1), 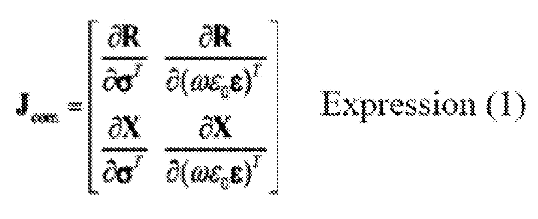
J_{com}: complex sensitivity matrix,
R: resistance component (real part of complex impedance),
X: reactance component (real part of complex impedance),
σ: conductivity,
ε: permittivity,
ω: angular frequency,
ε₀: vacuum permittivity, and
T: transpose.

3. The electrical tomography method according to Claim 2,
wherein the complex sensitivity matrix is a complex sensitivity matrix calculated using partial derivatives of a complex sensitivity matrix based on Geselowitz theory.

4. The electrical tomography method according to Claim 3,
wherein the complex sensitivity matrix is calculated using partial derivatives of a complex sensitivity matrix derived from Expression (2) that represents a relationship between complex impedance and complex potential field obtained by modifying the Geselowitz theory,
where in Expression (2), 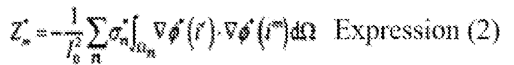 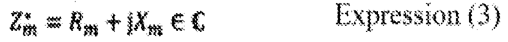 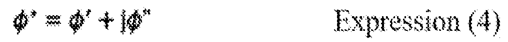 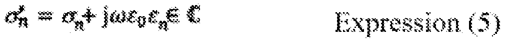 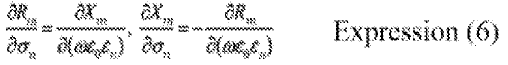 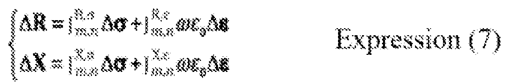 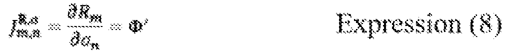 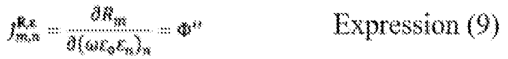 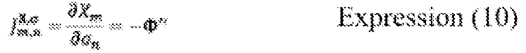 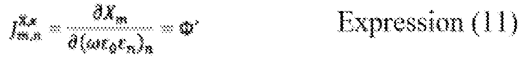 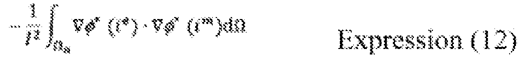
Zₘ*: complex impedance measured in m-th electrode pattern represented by Expression (3),
φ*: complex potential distribution represented by Expression (4),
σₙ*: complex conductivity of n-th mesh represented by Expression (5), and
Ωₙ: area of n-th mesh, in Expression (3),
Rₘ: resistance component of complex impedance measured in m-th electrode pattern, and
Xₘ: reactance component of complex impedance measured in m-th electrode pattern, in Expression (4),
φ': value of real part of potential, and
φ": value of imaginary part of potential, in Expression (5),
σₙ: conductivity of n-th mesh,
ω: angular frequency,
ε₀: vacuum permittivity, and
εₙ: permittivity of n-th mesh, and
in Expressions (3) to (5), j denotes an imaginary unit.

5. The electrical tomography method according to Claim 2,
wherein the complex sensitivity matrix is a complex sensitivity matrix calculated using partial derivatives derived from perturbations in the permittivity and the conductivity.

6. The electrical tomography method according to any one of Claims 1 to 5,
wherein a distribution of at least any one of permittivity and conductivity in a plurality of dimensions of the measurement object is visualized.

7. The electrical tomography method according to Claim 6,
wherein the plurality of dimensions are two or more dimensions selected from the group consisting of five dimensions of a frequency, a time, and a three-dimensional space.

8. The electrical tomography method according to Claim 1,
wherein the measurement object is a biologically derived substance or a non-biologically derived substance.

9. The electrical tomography method according to Claim 8,
wherein the biologically derived substance is any one or more selected from the group consisting of a cell, a tissue, a living body, and a biological constituent substance.

10. The electrical tomography method according to Claim 8,
wherein the non-biologically derived substance is any one or more selected from the group consisting of liquid containing bubbles, powder, slurry, and plastic.

11. The electrical tomography method according to any one of Claims 1 to 5,
wherein the measurement object is a cell or tissue, and
a concentration of ions flowing into and out of ion channels of the measurement object is visualized by applying stimulation to the measurement object by potential control from outside the measurement object.

12. The electrical tomography method according to Claim 11,
wherein a constant voltage or a periodically changing voltage is injected to the measurement object by a single electrode or a plurality of electrodes to control movement and activity of the measurement object.

13. An electrical tomography device comprising:
three or more electrodes;
a calculation unit configured to calculate a complex sensitivity matrix; and
a display unit configured to display at least any one of a distribution of permittivity and a distribution of conductivity of a measurement object based on the complex sensitivity matrix.

14. The electrical tomography device according to Claim 13,
wherein the complex sensitivity matrix is represented by Expression (1), 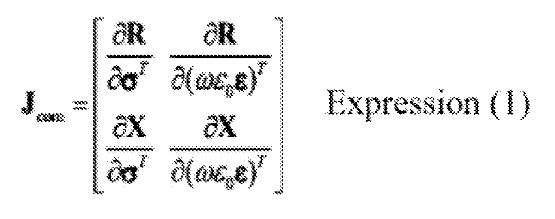 where in Expression (1),
J_{com}: complex sensitivity matrix,
R: resistance component (real part of complex impedance),
X: reactance component (real part of complex impedance),
σ: conductivity,
ε: permittivity,
ω: angular frequency,
ε₀: vacuum permittivity, and
T: transpose.

15. The electrical tomography device according to Claim 14,
wherein the calculation unit calculates the complex sensitivity matrix using partial derivatives of a complex sensitivity matrix based on Geselowitz theory.

16. The electrical tomography device according to Claim 15,
wherein the calculation unit derives the partial derivatives of the complex sensitivity matrix from Expression (2) that represents a relationship between complex impedance and complex potential field obtained by modifying the Geselowitz theory, 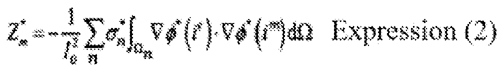 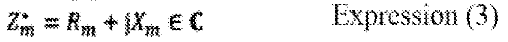 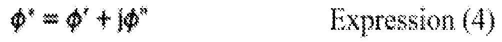 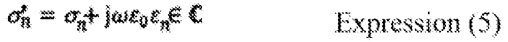 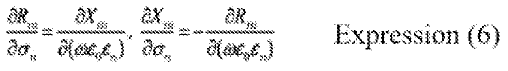 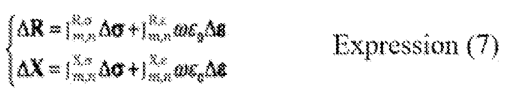 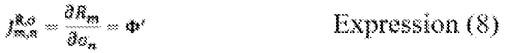 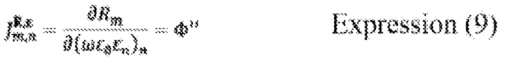 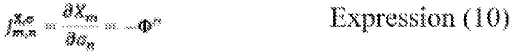 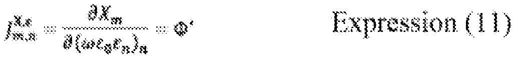 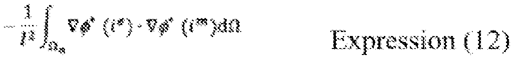 where in Expression (2),
Zₘ*: complex impedance measured in m-th electrode pattern represented by Expression (3),
φ*: complex potential distribution represented by Expression (4),
σₙ*: complex conductivity of n-th mesh represented by Expression (5), and
Ωₙ: area of n-th mesh, in Expression (3),
Rₘ: resistance component of complex impedance measured in m-th electrode pattern, and
Xₘ: reactance component of complex impedance measured in m-th electrode pattern, in Expression (4),
φ': value of real part of potential, and
φ": value of imaginary part of potential, in Expression (5),
σₙ: conductivity of n-th mesh,
ω: angular frequency,
ε₀: vacuum permittivity, and
εₙ: permittivity of n-th mesh, and
in Expressions (3) to (5), j denotes an imaginary unit.

17. The electrical tomography device according to Claim 14,
wherein the calculation unit calculates partial derivatives derived from perturbations in the permittivity and the conductivity, and calculates the complex sensitivity matrix from the partial derivatives.

18. The electrical tomography device according to any one of Claims 13 to 17,
wherein the display unit displays at least any one of a distribution of permittivity and a distribution of conductivity in a plurality of dimensions of the measurement object.

19. The electrical tomography device according to Claim 18,
wherein the plurality of dimensions are two or more dimensions selected from the group consisting of five dimensions of a frequency, a time, and a three-dimensional space.

20. The electrical tomography device according to Claim 13,
wherein the measurement object is a biologically derived substance or a non-biologically derived substance.

21. The electrical tomography device according to Claim 20,
wherein the biologically derived substance is any one or more selected from the group consisting of a cell, a tissue, a living body, and a biological constituent substance.

22. The electrical tomography device according to Claim 20,
wherein the non-biologically derived substance is any one or more selected from the group consisting of liquid containing bubbles, powder, slurry, and plastic.

23. The electrical tomography device according to any one of Claims 13 to 17, further comprising:
a potential control unit configured to control potentials of the electrodes,
wherein the measurement object is a cell or tissue,
the potential control unit applies stimulation to the measurement object by potential control from outside the measurement object, and
the display unit displays a distribution of a concentration of ions flowing into and out of ion channels of the measurement object.

24. The electrical tomography device according to Claim 23,
wherein the potential control unit injects a constant voltage or a periodically changing voltage to the measurement object by a single electrode or a plurality of electrodes to control movement and activity of the measurement object.
